# EUROPÄISCHE PATENTSCHRIFT

(11) **EP 3 574 372 B1**
(45) Veröffentlichungstag und Bekanntmachung des Hinweises auf die Patenterteilung: **15.07.2020**
(21) Anmeldenummer: 18704453.2
(22) Anmeldetag: 25.01.2018
(51) Int. Cl.: G02C 13/00, G03B 17/48

(54) **VORRICHTUNG ZUR BESTIMMUNG VON ZENTRIERPARAMETERN FÜR DIE BRILLENANPASSUNG**
DEVICE FOR DETERMINING CENTRING PARAMETERS FOR SPECTACLE ADAPTATION
DISPOSITIF DE DÉTERMINATION DES PARAMÈTRES DE CENTRAGE POUR L'ADAPTATION DE LUNETTES

(30) Priorität: 27.01.2017 EP 17153556
(43) Veröffentlichungstag der Anmeldung: 04.12.2019
(60) Teilanmeldung: 19189087.0
(73) Patentinhaber: Carl Zeiss Vision International GmbH, 73430 Aalen (DE); Carl Zeiss AG, 73447 Oberkochen (DE)
(72) Erfinder: ALVAREZ DIEZ, Cristina, 73447 Oberkochen (DE); BREUNINGER, Tobias, 72385 Riederich (DE); GAMPERLING, Michael, 89340 Leipheim (DE); SCHWARZ, Oliver, 73479 Ellwangen (DE); WIDULLE, Frank, 89233 Neu-Ulm (DE)
(74) Vertreter: Patentanwälte Bregenzer und Reule Partnerschaftsgesellschaft mbB
(86) Internationale Anmeldenummer: PCT/EP2018/051873
(87) Internationale Veröffentlichungsnummer: WO 2018/138216

(56) Entgegenhaltungen:
- EP-A1- 3 012 687
- EP-A2- 1 038 495
- WO-A1-2005/033793
- FR-A1- 3 024 911
- US-A1- 2003 081 173
- US-A1- 2007 285 528
- US-A1- 2010 239 135
- US-A1- 2015 304 530
- US-B1- 9 395 562

## Beschreibung

Die Erfindung betrifft eine Vorrichtung zur Bestimmung von Zentrierparametern für die Brillenanpassung gemäß Oberbegriff des Anspruchs 1.

Zentrierparameter werden benutzt, um Brillengläser korrekt in einer Brillenfassung anzuordnen bzw. zu zentrieren, so dass die Brillengläser in korrekter Position relativ zu den Augen der die Brille tragenden Person angeordnet sind. Dabei handelt es sich zum Teil um anatomische Parameter der betreffenden Person, wie beispielsweise den Pupillenabstand, zum Teil um rein fassungsspezifische Parameter wie die Fassungsscheibenbreite oder die Fassungsscheibenhöhe und zum Teil um Kombinationen aus anatomischen und fassungsspezifischen Parametern, wie beispielsweise den Hornhautscheitelabstand und die Durchblickshöhe. Einen Überblick über die gängigen Zentrierparameter gibt die DIN EN ISO 13666 vom Oktober 2013.

Es sind Vorrichtungen bekannt, mit denen eine automatische Bestimmung von Zentrierparametern möglich ist. So ist beispielsweise aus der EP 1 844 363 B2 eine solche Vorrichtung bekannt, bei der mittels zweier Kameras aus unterschiedlichen Blickrichtungen Bilder einer eine Brille bzw. Brillenfassung tragenden Person aufgenommen werden, so dass aus den aufgenommenen Bilddaten Zentrierparameter bestimmt werden können. Aus der US 2010/239135 A1 ist eine Vorrichtung mit den Merkmalen des Oberbegriffs des Anspruches 1 bekannt, die der Untersuchung von Kindern mit Schiefköpfigkeit dient und keine Zentrierparameter bestimmt.

Bei bekannten Vorrichtungen zur Bestimmung von Zentrierparametern besteht jedoch oft das Problem, dass die aus unterschiedlichen Blickrichtungen aufgenommenen Bilder in Ihrer Qualität und Verwertbarkeit schwanken.

Es ist daher Aufgabe der Erfindung, eine Vorrichtung der eingangs genannten Art derart weiterzubilden, dass bessere Bilder der Person, für die die Zentrierparameter bestimmt werden sollen, erzeugt werden können.

Diese Aufgabe wird erfindungsgemäß durch eine Vorrichtung mit den Merkmalen des Anspruches 1 gelöst. Vorteilhafte Weiterbildungen der Erfindung sind Gegenstand der abhängigen Ansprüche.

Ausgehend von der US 2010/239135 löst der Gegenstand des Anspruchs 1 die Aufgabe, eine bessere Ausleuchtung des Kopfs eines Probanden zu erzielen.

Erfindungsgemäß weist die Vorrichtung einen Kameraträger auf, der einen Innenraum teilweise umschließt, diesen aber nach oben, nach unten und zu einer Rückseite hin offen lässt. Ein Proband kann sich dann von der Rückseite her in den Innenraum bewegen. Der Innenraum wird durch den Kameraträger nach vorne und zu den Seiten begrenzt. Die Kameras sind auf den Innenraum gerichtet. Sie sind in einer Kameraanordnung angeordnet, die sich zwischen zwei freien Enden des Kameraträgers erstreckt. Vorzugsweise erstreckt sich eine konkave Biegung des Kameraträgers in derselben Richtung, in der sich die Kameraanordnung zwischen den freien Enden erstreckt. Dabei ist es möglich, eine kontinuierliche und konkave Biegung vorzusehen, dem Kameraträger beispielsweise in Draufsicht eine Kreisbogenform zu geben, oder aber einen eckigen Grundriss vorzusehen, so dass der Kameraträger eine Frontpartie und in einem Winkel von der Frontpartie abstehende Seitenpartien aufweist. Die Kameras der Kameraanordnung können sämtlich auf gleicher Höhe in einer Kamerareihe angeordnet oder in ihrer Höhe gegeneinander versetzt sein. Um den Innenraum gut auszuleuchten, weist der Kameraträger eine Beleuchtungseinrichtung auf, die sich vorzugsweise zwischen den freien Enden über eine Länge erstreckt, die mindestens der in einer Umfangsrichtung zwischen den freien Enden gemessenen Länge der Kameraanordnung entspricht. Zweckmäßig weist die Beleuchtungseinrichtung mindestens eine Lichtleiste oder Leuchtmittelreihe mit einer Vielzahl von Leuchtmitteln auf. Die Leuchtmittel sind in einer oder mehreren Reihen angeordnet. Die mindestens eine Lichtleiste mit ihrer Vielzahl von Leuchtmitteln, die vorzugsweise als LEDs ausgebildet sind, ermöglicht eine in hohem Maße gleichmäßige Ausleuchtung des Innenraums, auf den die Kameras gerichtet sind. Erfindungsgemäß ist vorgesehen, dass die Beleuchtungseinrichtung so ausgestaltet ist, dass die auf einer vorbestimmten Oberfläche im Innenraum, nämlich in einem Bereich einer mittig im Innenraum angeordneten Zylindermantelfläche mit 20 cm Durchmesser, der sich über eine Höhe von 20cm und einen Mittelpunktswinkel von 180 Grad erstreckt, gemessene Lichtintensität an jeder Stelle der Oberfläche um maximal +50% und -30% von einem vorgegebenen Basiswert abweicht. Dieser beträgt beispielsweise 1000 Lux, was als ausreichende Ausleuchtung angesehen wird. Dabei kann die Beleuchtungseinrichtung so eingerichtet sein, dass die Lichtintensität an jeder Stelle des vorgegebenen Bereichs einen vorgegebenen Mindestwert überschreitet und dann nicht zwingend insbesondere in Abhängigkeit von Umgebungslicht geregelt werden muss. Die Homogenität des Lichts im Innenraum wird weiter verbessert, wenn die Leuchtmittel ihr Licht durch mindestens ein großflächiges Fenster in den Innenraum emittieren. Reflexionen in den Pupillen sind dann, wenn sie überhaupt auftreten, sehr schwach und stören die Zentriermessung kaum. Alle Leuchtmittel weisen zudem vorzugsweise eine im wesentlichen einheitliche Farbtemperatur von ca. 4000 K +/- 1000 K auf.

Um die Ausleuchtung des Innenraums weiter zu verbessern, wird bevorzugt, dass die Beleuchtungseinrichtung eine erste, über der Kameraanordnung angeordnete, und eine zweite, unter der Kameraanordnung angeordnete Lichtleiste aufweist. Weiter wird bevorzugt, dass die erste und die zweite Lichtleiste jeweils nahe den freien Enden des Kameraträgers durch vertikal verlaufende weitere Lichtleisten miteinander verbunden sind. Die Kameraanordnung wird dann an der Innenfläche des Kameraträgers ringsum durch die Leuchtmittel der Lichtleisten umrahmt, welche sich bevorzugt über einen Mittelpunktswinkel von mindestens 180 Grad erstrecken. Der Abstand der oberen Lichtleiste von der unteren Lichtleiste wird vorzugsweise so gewählt, dass die oben erwähnte Homogenität der Ausleuchtung mit der Abweichung von maximal +50% und -30% vom Basiswert in einem Bereich gegeben ist, der eine Höhe von ca. 20cm hat. Die vertikal verlaufenden weiteren Lichtleisten oder Leuchtmittelreihen erhöhen die Lichtintensität im Bereich der Enden der ersten und der zweiten Lichtleiste oder Leuchtmittelreihe, wo von jenseits dieser Enden kein Lichteinfall kommt.

Um aussagefähige Bilddaten erzeugen zu können, wird bevorzugt, dass sich die Anordnung der Kameras über einen Mittelpunktswinkel von mindestens 150 Grad erstreckt bzw. dass die optischen Achsen der dem freien Ende des Kameraträgers am nächsten gelegenen Kameras einen Winkel von mindestens 150 Grad einschließen. Es ist dann möglich, Bilder des Probanden nicht nur im Wesentlichen von vorn, sondern auch von beiden Seiten aufzunehmen. Zudem wird bevorzugt, dass sich die freien Enden des Kameraträgers in einem Abstand von mindestens 20cm und vorzugsweise von mindestens 25cm zueinander befinden. Ein Proband mit durchschnittlich breitem Kopf kann sich dann bequem von der Rückseite in den Innenraum bewegen.

Zweckmäßig weist die Kameraanordnung eine ungerade Zahl von Kameras auf mit einer mittig angeordneten Frontkamera und bezüglich einer durch die optische Achse der Frontkamera verlaufenden Symmetrieebene symmetrisch angeordneten Seitenkameras. Je mehr Kameras die Kameraanordnung enthält, desto genauer können die Zentrierparameter bestimmt werden.

Erfindungsgemäß ist eine Steuer- oder Regeleinrichtung zum automatischen oder manuellen Steuern oder Regeln der Lichtintensität der Leuchtmittel in Abhängigkeit von der Helligkeit im Innenraum und vorzugsweise in Abhängigkeit von der mittels der Kameras detektierten Helligkeit vorgesehen. Dabei wird bevorzugt, dass Sektoren der Beleuchtungseinrichtung und/oder einzelne Leuchtmittel getrennt voneinander steuerbar oder regelbar sind. Dadurch wird der Tatsache Rechnung getragen, dass in der Praxis regelmäßig Fremdlicht auf den Probanden einfällt, beispielsweise durch ein Fenster des Raumes, in dem sich die Vorrichtung befindet. Um eine gleichmäßige Ausleuchtung des Probanden zu gewährleisten, kann es dann erforderlich sein, beispielsweise die dem Fenster zugewandte Gesichtshälfte weniger stark, die dem Fenster abgewandte Gesichtshälfte aber umso stärker auszuleuchten.

Eine weitere vorteilhafte Weiterbildung oder alternative Ausgestaltung der Erfindung sieht einen Abstandssensor vor, der der Messung des Abstands des Kopfes des Probanden zur Mitte des Kameraträgers dient. Zudem ist eine Anzeigeeinheit zur Anzeige des Abstands oder einer den Abstand charakterisierenden Größe vorgesehen. Dadurch wird dem Probanden eine Positionierung am gewünschten Ort, beispielsweise in der Mitte des Innenraums, erleichtert. Während der Proband eine Positionierung in der optischen Achse der in der Mitte des Kameraträgers angeordneten Frontkamera meist selbst hinreichend genau vornehmen kann, kann er den Abstand zur Mitte des Kameraträgers meist nur schlecht abschätzen. Der Abstandssensor ermittelt diesen Abstand und zeigt dem Probanden an, ob er zu weit vorne, zu weit hinten oder richtig steht. Dabei kann ein zusätzlicher Abstandssensor vorgesehen sein, oder einige der Kameras bilden derart den Abstandssensor, dass sie aus unterschiedlichen Winkeln den Probanden aufnehmen und durch ein geeignetes Abstandsmessverfahren den Abstand bestimmen. Zudem kann die Anzeigeeinheit als zusätzliche Einheit ausgebildet sein und beispielsweise den Abstand als Abstandswert auf einem Display oder mittels Leuchtmitteln in unterschiedlichen Farben anzeigen. Bei letzterer Methode ist es möglich, beidseits eines grünen Leuchtmittels, das bei richtiger Positionierung aufleuchtet, je ein beispielsweise gelbes und/oder oranges und/oder rotes Leuchtmittel anzuordnen, das dann jeweils eine mehr oder weniger starke Abweichung von der richtigen Postionierung nach vorne bzw. hinten durch Aufleuchten anzeigt. Diese Art der Anzeige ist sehr anschaulich. Es wird jedoch bevorzugt, dass die Anzeigeeinheit durch die Beleuchtungseinrichtung gebildet wird. Diese kann so eingerichtet sein, dass sie die Farbe des abgestrahlten Lichts abhängig vom Abstand des Kopfes von der Mitte des Kameraträgers ändert und ausgehend von rein weißem Licht, das eine korrekte Positionierung anzeigt, immer mehr farbiges Licht beimischt. Es ist auch möglich, dass bei falscher Positionierung des Probanden die Beleuchtungseinrichtung durch örtliche Intensitäts- oder Farbänderungen ein Lauflicht erzeugt, das die Richtung anzeigt, in die sich der Proband zur richtigen Positionierung bewegen muss.

Vorteilhaft ist am Kameraträger eine Fixationseinrichtung zum Erzeugen eines Fixationsmusters für den Probanden angeordnet, vorzugsweise eine zumindest ein Specklemuster als Fixationsmuster erzeugende Fixationseinrichtung. Eine solche Fixationseinrichtung ist ausführlich in der EP 1 704 437 B1 beschrieben, auf die in diesem Zusammenhang ausdrücklich Bezug genommen wird.

Zweckmäßig wird mindestens einer der folgenden Zentrierparameter bestimmt: Hornhautscheitelabstand mindestens eines Auges, Durchblickpunkt durch die Ebene des Brillenglases für mindestens ein Auge, Abstand zwischen den Pupillen, Abstand mindestens einer der Pupillen zu einer Symmetrieebene der Brille oder Brillenfassung, Lage der Glasränder bezüglich mindestens einer Pupille, Verkippung der Fassungsebenen zur Vertikalen (pantoskopischer Winkel). Das erfindungsgemäße Verfahren kann dadurch vereinfacht werden, dass die Glasränder durch Boxen angenähert werden, die durch Boxlänge und Boxhöhe definiert werden.

Die erfindungsgemäß bestimmten Zentrierparameter können vorteilhaft zum Zentrieren eines Brillenglases in einer Brillenfassung und/oder zum Einschleifen eines Brillenglases in eine Brillenfassung herangezogen werden. Dabei wird in einem Verfahrensschritt das mindestens eine Brillenglas mit den bestimmten Zentrierparametern in der Brillenfassung zentriert oder es wird das mindestens eine Brillenglas basierend auf den bestimmten Zentrierparametern für eine Anordnung in der Brillenfassung eingeschliffen. Auf diese Weise können Brillengläser und Brillen hergestellt werden.

Im Folgenden wird die Erfindung anhand eines in der Zeichnung schematisch dargestellten Ausführungsbeispiels näher erläutert. Es zeigt:

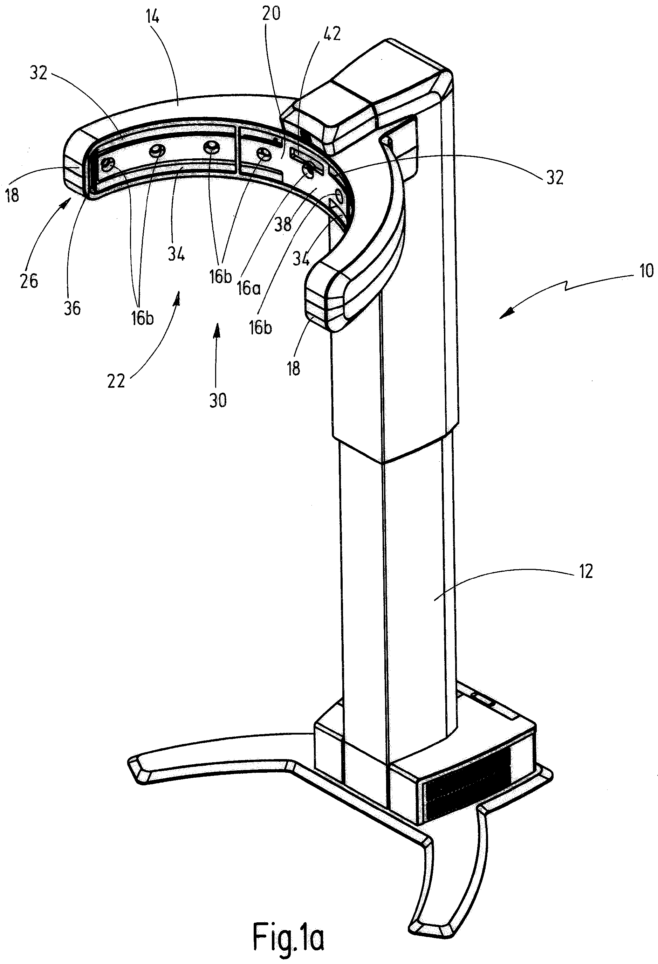

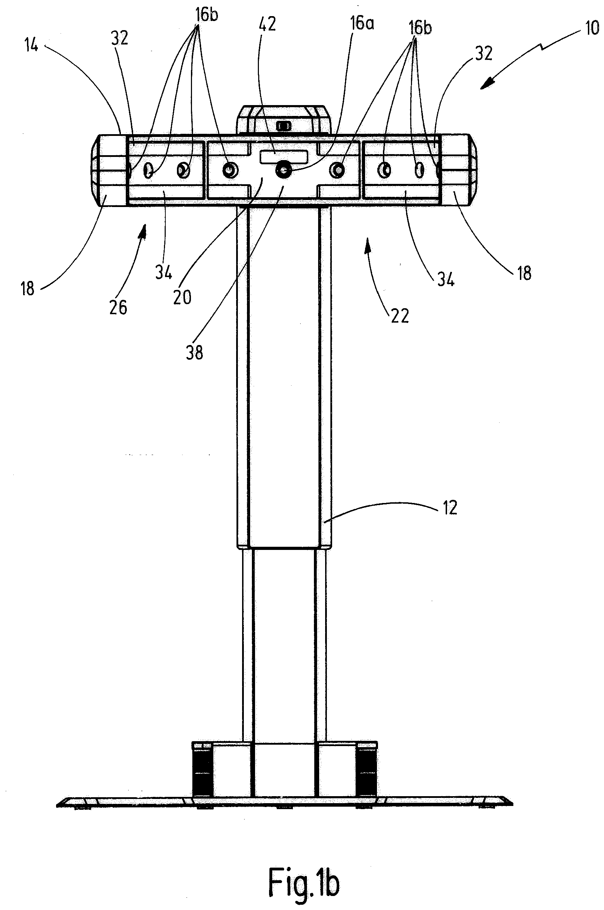

- Figur 1a, b: eine Vorrichtung zur Bestimmung von Zentrierparametern in perspektivischer Ansicht und in einer Ansicht von vorne.

Die in der Zeichnung dargestellte Vorrichtung 10 dient der Bestimmung von Zentrierparametern für die Brillenanpassung. Sie weist eine Säule 12 auf, die höhenverstellbar einen starren Kameraträger 14 trägt, welcher wiederum eine Anzahl Kameras 16a, 16b trägt. Der Kameraträger 14 ist in Draufsicht näherungsweise kreisförmig gebogen und erstreckt sich zwischen zwei freien Enden 18, welche im Abstand zueinander angeordnet sind. Nach vorne, also zur Säule 12 hin, und zu den Seiten umschließt eine Innenfläche 20 des Kameraträgers 14 einen Innenraum 22, in dem sich bei der Aufnahme von Bildern durch die Kameras 16a, 16b der Kopf eines Probanden befindet. Die Innenfläche 20 ist in einer Richtung, die zwischen den freien Enden 18 verläuft, konkav gebogen und weist beispielsweise die Form eines Abschnitts einer Zylindermantelfläche auf, wobei der Zylinder eine kreisrunde oder ovale Grundfläche haben kann. Um den Kameraträger 14 bezüglich des Kopfs des Probanden auf der richtigen Höhe positionieren zu können, ist in der Säule 12 eine nicht näher dargestellte Hubeinrichtung angeordnet, mit der der Kameraträger 14 motorisch angetrieben auf und ab bewegt werden kann.

Alle Kameras 16a, 16b sind äquiangular in einer sich zwischen den freien Enden 18 erstreckenden Kameraanordnung 26 angeordnet. Im vorliegenden Ausführungsbeispiel ist die Kameraanordnung 26 als Kamerareihe 26 ausgebildet, deren Kameras 16a, 16b sich alle in derselben Höhe befinden, wobei ihre optischen Achsen auf den Innenraum 22 gerichtet sind. Im vorliegenden Ausführungsbeispiel umfasst die Kamerareihe 26 eine in der Mitte des Kameraträgers 14 angeordnete Frontkamera 16a, deren optische Achse frontal auf das Gesicht des Probanden gerichtet ist, sowie acht paarweise symmetrisch bezüglich einer durch die optische Achse der Frontkamera 16a verlaufenden senkrechten Symmetrieebene angeordnete Seitenkameras 16b von denen jeweils vier von links und von rechts auf das Gesicht des Probanden gerichtet sind. Die Kameras 16a, 16b sind zudem kalibriert, so dass sie gleichzeitig kalibrierte Bilder des Probanden aufnehmen können. Die Kalibrierung umfasst die extrinsischen Eigenschaften wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird. Eine ausführliche Beschreibung der Kalibrierung von Kameras findet sich im Lehrbuch "Multiple View Geometry in Computer Vision" von Richard Hartley und Andrew Zisserman, 2. Auflage, Cambridge University Press 2004, und dort insbesondere auf Seite 8.

Der Kameraträger 14 umschließt den Innenraum 22 nur nach vorne, zur Säule 12 hin, und zu den Seiten, also links und rechts des Kopfes des Probanden. Nach oben, unten sowie zu einer Rückseite 30 hin ist er offen, wobei die freien Enden 18 zueinander einen Abstand von mindestens 25cm aufweisen, so dass sich der Proband bequem von der Rückseite aus nähern kann. Im gezeigten Ausführungsbeispiel beträgt der Abstand 70 bis 80cm, und die Kameras 16a, 16b sind in gleichen Winkelabständen zueinander angeordnet.

Zur Ausleuchtung des Innenraums 22 ist eine Beleuchtungseinrichtung mit einer oberhalb der Kamerareihe 26 verlaufenden oberen Lichtleiste 32 sowie einer unterhalb der Kamerareihe 26 verlaufenden unteren Lichtleiste 34 vorgesehen, welche jeweils eine Vielzahl von LEDs als Leuchtmittel aufweisen. Die obere Lichtleiste 32 und die untere Lichtleiste 34 erstrecken sich jeweils durchgehend oder mit Unterbrechungen über eine Länge, die mindestens so groß ist wie die Länge der in Umfangsrichtung zwischen den freien Enden 18 gemessenen Länge der Kamerareihe 26. Diese entspricht einem Umfangswinkel von mindestens 160 Grad. Nahe den freien Enden 18 sind die obere Lichtleiste 32 und die untere Lichtleiste 34 jeweils mittels einer vertikal verlaufenden weiteren Lichtleiste 36 miteinander verbunden. Die Kamerareihe 26 wird somit vollständig durch mindestens eine Reihe LEDs umrahmt. Die Vorrichtung 10 weist zudem eine in der Zeichnung nicht näher dargestellte Steuer- oder Regeleinrichtung auf, mit der die von den LEDs abgestrahlte Lichtintensität abhängig von der durch die Kameras 16a, 16b detektierten Lichtintensität gesteuert oder geregelt werden kann. Die LEDs der Lichtleisten 32, 34, 36 sind dabei zu Sektoren zusammengefasst, deren abgestrahlte Lichtintensitäten getrennt voneinander gesteuert bzw. geregelt werden können. Zudem ist vorgesehen, dass auch die von den einzelnen LEDs abgestrahlten Lichtintensitäten mittels der Steuer- oder Regeleinrichtung getrennt voneinander gesteuert oder geregelt werden können.

Um den Probanden richtig im Innenraum 22 positionieren zu können, sind die beiden der Frontkamera 16a nächstgelegenen Seitenkameras 16b dazu eingerichtet, den Abstand des Kopfs des Probanden von der Mitte 38 des Kameraträgers 14 zu messen. Mittels einer nicht näher dargestellten Anzeigeeinheit wird dem Probanden angezeigt, ob er richtig steht oder nicht. Die Anzeigeeinheit weist mehrere unterschiedlich eingefärbte Lichtquellen auf, die in einer Reihe angeordnet sind. Die mittlere Lichtquelle leuchtet grün, wenn der Proband richtig steht. Ausgehend von der mittleren Lichtquelle gibt es in jeder Richtung in dieser Reihenfolge eine gelbe, eine orangene und eine rote Lichtquelle, die entsprechend der Farbe anzeigt, wenn der Proband ein wenig, deutlich oder viel zu weit von der Mitte 38 des Kameraträgers 14 entfernt bzw. ein wenig, deutlich oder viel zu nah zur Mitte 38 steht. Um bei der Bestimmung der Zentrierparameter sicherzustellen, dass die Blickrichtung des Probanden ins Unendliche gerichtet ist, ist eine am Kameraträger 14 angeordnete Fixationseinrichtung 42 vorgesehen, die ein Fixationsmuster für den Probanden in Form eines Specklemusters erzeugt. Das Fixationsmuster ist etwas höher angeordnet als die Frontkamera 16a, so dass der Proband über diese hinwegblickt. Damit kann sein Gesicht im größtmöglichen Umfang aufgenommen werden.

Die Vorrichtung 10 eignet sich insbesondere auch zur Herstellung eines Avatars des Kopfs des Probanden, welcher zur Bestimmung der Zentrierparameter herangezogen werden kann. Zum diesem Zweck werden durch die Kameras 16a, 16b kalibrierte Bilder des Kopfs des Probanden ohne Brille bzw. Brillenfassung aufgenommen. Dabei ist es vorteilhaft, wenn die Bilder gleichzeitig aufgenommen werden. Mittels eines geeigneten Prozesses zur geometrischen Positionsbestimmung wie beispielsweise Triangulation, Stereo-Rekonstruktion, Multiview-Reconstruction oder Structure from Motion wird ein dreidimensionales Tiefenprofil des Kopfes erstellt, das diesen näherungsweise sehr gut abbildet. Der Kopf wird durch eine Vielzahl von Punkten abgebildet, die mittels eines Netzmusters miteinander verbunden werden können oder aber als Punktwolke gespeichert werden können. Bei der anschließenden Bestimmung der Zentrierparameter kann der so ermittelte Avatar herangezogen werden, um Zentrierparameter zu bestimmen, die aufgrund der geometrischen Eigenschaften der Brille bzw. Brillenfassung, die der Proband trägt, nicht oder nur näherungsweise bestimmt werden können. Beispielsweise kann ein breiter Fassungsbügel das Auge in einer Seitenaufnahme soweit verdecken, dass der Hornhautscheitelabstand nicht oder nur sehr ungenau bestimmt werden kann. Zudem können gefärbte oder stark spiegelnde Gläser die Augen nicht oder nur sehr schlecht erkennen lassen. Um dem zu begegnen, wird auf die von den Kameras 16a, 16b aufgenommenen Bilder des die Brille oder Brillenfassung tragenden Probanden das Tiefenprofil des Avatars projiziert und die Zentrierparameter, die aufgrund der durch die Brille bzw. Brillenfassung eingeschränkten Sicht nur ungenügend bestimmt werden können, werden mittels der Bilddaten des Avatars bestimmt. Dabei kann eine Anpassung des Avatars auf die Bilder des die Brille bzw. Brillenfassung tragenden Probanden zur Minimierung von Abweichungen erfolgen. Zudem kann der Avatar dazu herangezogen werden, virtuell Brillen bzw. Brillenfassungen anzuprobieren, indem die ihre Geometrie beschreibenden Daten auf den Avatar eingeblendet werden.

## Patentansprüche

1. Vorrichtung zur Bestimmung von Zentrierparametern mit einem Kameraträger (14), welcher einen nach oben, nach unten und zu einer Rückseite (30) hin offenen Innenraum (22) teilweise umschließt und mindestens drei Kameras (16a, 16b) trägt, die zwischen zwei freien Enden (18) des Kameraträgers (14) angeordnet und auf den Innenraum (22) gerichtet sind, wobei der Kameraträger (14) zur Beleuchtung des Innenraums (22) eine Beleuchtungseinrichtung (32, 34, 36) aufweist, **dadurch gekennzeichnet, dass** die Beleuchtungseinrichtung (32, 34, 36) eingerichtet ist, den Innenraum (22) so auszuleuchten, dass die Lichtintensität zumindest an jeder Stelle eines Bereichs, der sich über eine Höhe von 20cm und einen Mittelpunktswinkel von 180 Grad einer im Innenraum (22) angeordneten Zylindermantelfläche mit 20 cm Durchmesser erstreckt, um maximal +50% und -30% von einem vorgegebenen Sollwert von 1000 Lux abweicht und dass eine Steuer- oder Regeleinrichtung zum Steuern oder Regeln der Lichtintensität der Leuchtmittel in Abhängigkeit von der Helligkeit im Innenraum (22) unter Berücksichtigung eines Fremdlichteinfalls vorgesehen ist.

2. Vorrichtung nach Anspruch 1, **dadurch gekennzeichnet, dass** Sektoren der Beleuchtungseinrichtung (32, 34, 36) und/oder einzelne Leuchtmittel getrennt voneinander steuerbar oder regelbar sind.

3. Vorrichtung nach einem der vorangehenden Ansprüche, **gekennzeichnet durch** einen Abstandssensor zur Messung des Abstands des Kopfes eines Probanden zur Mitte des Kameraträgers (14) sowie eine Anzeigeeinheit zur Anzeige des Abstands oder einer den Abstand charakterisierenden Größe.

4. Vorrichtung nach einem der vorangehenden Ansprüche, **dadurch gekennzeichnet, dass** die Homogenität des Lichts im Innenraum weiter verbessert wird, wenn die Leuchtmittel ihr Licht durch mindestens ein großflächiges Fenster in den Innenraum emittieren.

5. Vorrichtung nach einem der vorangehenden Ansprüche, **dadurch gekennzeichnet, dass** die die Beleuchtungseinrichtung (32, 34, 36) so eingerichtet ist, dass die Lichtintensität an jeder Stelle des Bereichs einen vorgegebenen Mindestwert überschreitet.

6. Vorrichtung nach einem der vorangehenden Ansprüche, **dadurch gekennzeichnet, dass** die Kameras in einer sich zwischen den freien Enden (18) des Kameraträgers (14) erstreckenden Kameraanordnung (26) angeordnet sind und dass sich die Beleuchtungseinrichtung (32, 34, 36) zwischen den freien Enden (18) über eine Länge erstreckt, die mindestens der Länge der Kameraanordnung (26) entspricht.

7. Vorrichtung nach Anspruch 6, **dadurch gekennzeichnet, dass** die Beleuchtungseinrichtung (32, 34, 36) mindestens eine Lichtleiste oder eine Leuchtmittelreihe mit einer Vielzahl von Leuchtmitteln aufweist.

8. Vorrichtung nach Anspruch 7, **dadurch gekennzeichnet, dass** die Beleuchtungseinrichtung eine erste, über der Kameraanordnung (26) angeordnete, und eine zweite, unter der Kameraanordnung (26) angeordnete Lichtleiste (32, 34) oder Leuchtmittelreihe aufweist.

9. Vorrichtung nach Anspruch 8, **dadurch gekennzeichnet, dass** die Beleuchtungseinrichtung die erste und die zweite Lichtleiste (32, 34) oder Leuchtmittelreihe jeweils nahe den freien Enden (18) verbindende, vertikal verlaufende weitere Lichtleisten (36) oder Leuchtmittelreihen aufweist.

10. Vorrichtung nach einem der vorangehenden Ansprüche, **dadurch gekennzeichnet, dass** sich die Anordnung der Kameras (16a, 16b) über einen Mittelpunktswinkel von mindestens 150 Grad erstreckt und/oder dass die optischen Achsen der den freien Enden (18) des Kameraträgers (14) am nächsten gelegenen Kameras (16b) einen Winkel von mindestens150 Grad einschließen.

11. Vorrichtung nach einem der vorangehenden Ansprüche, **dadurch gekennzeichnet, dass** die freien Enden (18) des Kameraträgers (14) in einem Abstand von mindestens 25cm zueinander angeordnet sind.

12. Verwendung einer Vorrichtung (10) nach einem der Ansprüche 1 bis 11 zur Durchführung eines Verfahrens zur Bestimmung von Zentrierparametern, bei dem ein Kopf eines eine Brille tragenden Probanden aus mindestens drei Aufnahmerichtungen aufgenommen wird und bei dem der Kopf des Probanden derart beleuchtet wird, dass die Beleuchtungsstärke an jeder Stelle des Kopfes, die aufgenommen wird, um maximal +50% und -30% von einem vorgegebenen Sollwert abweicht.

## Claims

1. Apparatus for determining centration parameters, comprising a camera carrier (14), which partly encloses an interior (22) that is open to the top, to the bottom and to a rear side (30) and which carries at least three cameras (16a, 16b) which are arranged between two free ends (18) of the camera carrier (14) and directed onto the interior (22), wherein the camera carrier (14) has an illumination device (32, 34, 36) for illuminating the interior (22), **characterized in that** the illumination device (32, 34, 36) is configured to fully illuminate the interior (22) such that the light intensity deviates from a specified target value of 1000 lux by at most +50% and -30% at least at every point of a region extending over a height of 20 cm and a centre angle of 180 degrees of a lateral surface of a cylinder, arranged in the interior (22), with a diameter of 20 cm, and **in that** an open-loop or closed-loop control device is provided for controlling or regulating the light intensity of the light-emitting means in dependence on the brightness in the interior (22) taking into account an incidence of extraneous light.

2. Apparatus according to Claim 1, **characterized in that** sectors of the illumination device (32, 34, 36) and/or individual light-emitting means are controllable or regulatable separately from one another by open-loop or closed-loop control.

3. Apparatus according to either of the preceding claims, **characterized by** a distance sensor for measuring the distance of the head of a subject from the centre of the camera carrier (14) and also a display unit for indicating the distance or a variable characterizing the distance.

4. Apparatus according to one of the preceding claims, **characterized in that** the homogeneity of the light in the interior is improved further if the light-emitting means emit their light into the interior through at least one large-area window.

5. Apparatus according to one of the preceding claims, **characterized in that** the illumination device (32, 34, 36) is configured such that the light intensity at each point of the region exceeds a specified minimum value.

6. Apparatus according to one of the preceding claims, **characterized in that** the cameras are arranged in a camera arrangement (26) extending between the free ends (18) of the camera carrier (14) and **in that** the illumination device (32, 34, 36) extends between the free ends (18) over a length that corresponds to at least the length of the camera arrangement (26).

7. Apparatus according to Claim 6, **characterized in that** the illumination device (32, 34, 36) has at least one light strip or a light-emitting means row having a multiplicity of light-emitting means.

8. Apparatus according to Claim 7, **characterized in that** the illumination device has a first light strip (32) or light-emitting means row arranged above the camera arrangement (26) and a second light strip (34) or light-emitting means row arranged below the camera arrangement (26) .

9. Apparatus according to Claim 8, **characterized in that** the illumination device has further vertically extending light strips (36) or light-emitting means rows that connect the first and the second light strip (32, 34) or light-emitting means row in each case near the free ends (18).

10. Apparatus according to one of the preceding claims, **characterized in that** the arrangement of the cameras (16a, 16b) extends over a centre angle of at least 150 degrees and/or **in that** the optical axes of the cameras (16b) located closest to the free ends (18) of the camera carrier (14) enclose an angle of 150 degrees.

11. Apparatus according to one of the preceding claims, **characterized in that** the free ends (18) of the camera carrier (14) are arranged at a distance of at least 25 cm in relation to one another.

12. Use of an apparatus (10) according to one of Claims 1 to 11 for carrying out a method for determining centration parameters, in which the head of a subject wearing spectacles is recorded from at least three recording directions and in which the head of the subject is illuminated such that the illuminance at every point of the head that is recorded deviates from a specified target value by at most +50% and -30%.

## Revendications

1. Dispositif permettant de déterminer des paramètres de centrage, comportant un support de caméras (14) qui entoure partiellement un espace intérieur (22) ouvert vers le haut, vers le bas et en direction d'une face arrière (30) et qui porte au moins trois caméras (16a, 16b) qui sont agencées entre deux extrémités libres (18) du support de caméras (14) et orientées vers l'espace intérieur (22), dans lequel le support de caméras (14) comporte un dispositif d'éclairage (32, 34, 36) afin d'éclairer l'espace intérieur (22), **caractérisé en ce que** le dispositif d'éclairage (32, 34, 36) est conçu pour éclairer l'espace intérieur (22) de telle manière que l'intensité lumineuse, au moins en tout point d'une zone qui s'étend sur une hauteur de 20 cm et à un angle au centre de 180 degrés d'une surface extérieure cylindrique ayant un diamètre de 20 cm agencée dans l'espace intérieur (22), s'écarte au maximum de +50 % et de -30 % d'une valeur de consigne prédéterminée de 1000 lux, et **en ce qu'**il est prévu un dispositif de commande ou de régulation servant à commander ou à réguler l'intensité lumineuse des moyens d'éclairage en fonction de la luminosité dans l'espace intérieur (22) en tenant compte d'une incidence de lumière parasite.

2. Dispositif selon la revendication 1, **caractérisé en ce que** des secteurs du dispositif d'éclairage (32, 34, 36) et/ou des moyens d'éclairage individuels peuvent être commandés ou régulés séparément les uns des autres.

3. Dispositif selon l'une des revendications précédentes, **caractérisé par** un capteur de distance servant à mesurer la distance de la tête d'une personne testée par rapport au centre du support de caméras (14) ainsi que par une unité d'affichage servant à afficher la distance ou une grandeur caractérisant la distance.

4. Dispositif selon l'une des revendications précédentes, **caractérisé en ce que** l'homogénéité de la lumière dans l'espace intérieur est encore améliorée lorsque les moyens d'éclairage émettent leur lumière dans l'espace intérieur à travers au moins une fenêtre de grande surface.

5. Dispositif selon l'une des revendications précédentes, **caractérisé en ce que** le dispositif d'éclairage (32, 34, 36) est conçu de telle manière que l'intensité lumineuse en tout point de la zone dépasse une valeur minimale prédéterminée.

6. Dispositif selon l'une des revendications précédentes, **caractérisé en ce que** les caméras sont disposées selon un agencement de caméras (26) qui s'étend entre les extrémités libres (18) du support de caméras (14) et **en ce que** le dispositif d'éclairage (32, 34, 36) s'étend entre les extrémités libres (18) sur une longueur qui correspond au moins à la longueur de l'agencement de caméras (26).

7. Dispositif selon la revendication 6, **caractérisé en ce que** le dispositif d'éclairage (32, 34, 36) comporte au moins une bande lumineuse ou une rangée de moyens d'éclairage comprenant une pluralité de moyens d'éclairage.

8. Dispositif selon la revendication 7, **caractérisé en ce que** le dispositif d'éclairage comporte une première bande lumineuse (32, 34) ou rangée de moyens d'éclairage disposée au-dessus de l'agencement de caméras (26) et une deuxième bande lumineuse ou rangée de moyens d'éclairage disposée en dessous de l'agencement de caméras (26).

9. Dispositif selon la revendication 8, **caractérisé en ce que** le dispositif d'éclairage comporte d'autres bandes lumineuses (36) ou rangées de moyens d'éclairage s'étendant verticalement et reliant respectivement les première et deuxième bandes lumineuses (32, 34) ou rangées de moyens d'éclairage à proximité des extrémités libres (18).

10. Dispositif selon l'une des revendications précédentes, **caractérisé en ce que** l'agencement des caméras (16a, 16b) s'étend sur un angle au centre d'au moins 150 degrés et/ou
**en ce que** les axes optiques des caméras (16b) situées le plus près des extrémités libres (18) du support de caméras (14) forment un angle d'au moins 150 degrés.

11. Dispositif selon l'une des revendications précédentes, **caractérisé en ce que** les extrémités libres (18) du support de caméras (14) sont agencées à une distance d'au moins 25 cm l'une de l'autre.

12. Utilisation d'un dispositif (10) selon l'une des revendications 1 à 11 pour la mise en œuvre d'un procédé permettant de déterminer des paramètres de centrage, selon lequel la tête d'une personne portant des lunettes est acquise à partir d'au moins trois directions d'acquisition et selon lequel la tête de la personne testée est éclairée de telle manière que l'intensité d'éclairement acquise en tout point de la tête s'écarte au maximum de +50% et de -30% d'une valeur de consigne prédéterminée.
